# EUROPEAN PATENT APPLICATION

(11) **EP 4 834 238 A1**
(43) Date of publication of application: **04.11.2026**
(21) Application number: 26175300.8
(22) Date of filing: 29.04.2026
(51) Int. Cl.: A61B 8/12, A61B 1/00, A61B 8/00

(54) **ULTRASOUND PROBE**

(30) Priority: 02.05.2025 JP 2025076759
(71) Applicant: FUJIFILM Corporation, Tokyo 106-8620 (JP)
(72) Inventor: UI, Kento, Tokyo (JP); NISHIMURA, Shogo, Tokyo (JP); TAKAHASHI, Tomoo, Tokyo (JP)
(74) Representative: Parker, Andrew James

(57) **Abstract**

Provided is an ultrasound probe having good operability.

An ultrasound probe includes an operation unit. The operation unit has a bulged portion 26 and a constricted portion. The constricted portion has a constricted surface having a saddle shape. In the constricted surface, a front slope is steeper than a rear slope. A back and a belly are divided by a pair of ridges.

## Description

### BACKGROUND OF THE INVENTION

### 1. Field of the Invention

The present disclosure relates to an ultrasound probe, and particularly to an ultrasound probe that is inserted into a body cavity.

### 2. Description of the Related Art

In an ultrasound examination of a living body, an ultrasound probe is used. As the intracavitary insertion-type ultrasound probe, an ultrasound probe that is inserted into a vagina, an ultrasound probe that is inserted into a rectum, and the like are known. Hereinafter, the intracavitary insertion-type ultrasound probe will be simply referred to as an ultrasound probe.

The ultrasound probe generally includes a head, a shaft, and an operation unit. The head includes an ultrasound transducer. The head is held by the shaft. The shaft is supported by the operation unit. The operation unit is a non-insertion part that is held by a hand of a user (a doctor, an examination technician, or the like).

JP2019-37606A (FIG. 1) and JP2019-37607A (FIG. 1) disclose the intracavitary insertion-type ultrasound probe. The operation unit included in the ultrasound probe has a substantially rod-like or cylindrical form as a whole.

### SUMMARY OF THE INVENTION

In the ultrasound examination using the ultrasound probe, after the head is inserted into the body cavity, a posture or a position of the operation unit is changed, for example, in a state in which the operation unit is lightly pinched by a plurality of fingers, and thus a posture or a position of the head is changed. In this process, it is desired that a holding state of the operation unit is stably generated. In a case in which a wave transmitting/receiving surface of the head is brought into close contact with a tissue surface in the body cavity, it is desired that a force is naturally transmitted from the plurality of fingers to the operation unit without causing pain or discomfort to the examinee.

The ultrasound examination is performed in a dark room. In the ultrasound examination, the user usually gazes at the ultrasound image. In such a situation, it is desired that a rotation angle (rotation angle around a shaft central axis) of the ultrasound probe can be reliably recognized through a tactile sense of the plurality of fingers pinching the operation unit.

An object of the present disclosure is to improve operability of an ultrasound probe. Alternatively, an object of the present disclosure is to provide an ultrasound probe comprising an operation unit that is easily placed on a finger, that is easily hooked by the finger, or that allows a rotation angle to be naturally recognized through a tactile sense of the finger.

An ultrasound probe according to the present disclosure is an ultrasound probe having a z direction that is a front-rear direction, an x direction that is a left-right direction orthogonal to the z direction, and a y direction that is an up-down direction orthogonal to the z direction and the x direction, the ultrasound probe comprising: a head that has an ultrasound transducer and is inserted into a body cavity; a shaft provided on a rear side of the head, supporting the head, and extending in the z direction; and an operation unit provided on a rear side of the shaft and supporting the shaft, in which the operation unit has a back facing upward and a belly facing downward, the belly has a constricted surface recessed upward as viewed in the x direction, the constricted surface has a saddle shape including a front slope and a rear slope, and the front slope is steeper than the rear slope as viewed in the x direction.

According to the present disclosure, it is possible to improve the operability of the ultrasound probe. Alternatively, according to the present disclosure, it is possible to provide an ultrasound probe comprising an operation unit that is easily placed on a finger, that is easily hooked by the finger, or that allows a rotation angle to be naturally recognized through a tactile sense of the finger.

### BRIEF DESCRIPTION OF THE DRAWINGS

FIG. 1 is a front view showing an ultrasound probe according to an embodiment.
FIG. 2 is an enlarged view showing a head.
FIG. 3 is a perspective view showing the ultrasound probe according to the embodiment.
FIG. 4 is an enlarged view showing an operation unit.
FIG. 5 is a perspective view showing a belly.
FIG. 6 is a schematic view showing a constricted surface.
FIG. 7 is a plan view showing the ultrasound probe according to the embodiment.
FIG. 8 is a diagram showing a plurality of cross-section positions.
FIG. 9 is a diagram showing a plurality of xy cross-sections.
FIG. 10 is a diagram showing an ultrasound probe to which a puncture adapter is attached.
FIG. 11 is a front view showing an attachment portion.
FIG. 12 is a cross-sectional view showing the attachment portion.
FIG. 13 is a diagram showing an ultrasound probe according to a modification example.

### DESCRIPTION OF THE PREFERRED EMBODIMENTS

Hereinafter, an embodiment will be described with reference to the accompanying drawings.

### (1) Outline of embodiment

For an ultrasound probe according to the embodiment, a z direction which is a front-rear direction, an x direction which is a left-right direction orthogonal to the z direction, and a y direction which is an up-down direction orthogonal to the z direction and the x direction are defined.

An ultrasound probe according to the embodiment includes a head, a shaft, and an operation unit. The head is a portion that includes an ultrasound transducer and is inserted into a body cavity. The shaft is a portion provided on a rear side of the head, supports the head, and extends in the z direction. The operation unit is a portion provided on a rear side of the shaft and supporting the shaft. The operation unit includes a back that faces upward and a belly that faces downward. The belly has a constricted surface that is recessed upward as viewed from the x direction. The constricted surface has a saddle shape including a front slope and a rear slope. The front slope is steeper than the rear slope as viewed from the x direction.

In the above-described configuration, in a case where the operation unit is held by a hand of a user, for example, the constricted surface is supported by a finger or the finger is wound around the constricted surface. Since the constricted surface has a saddle shape (which can also be said to be a reversed saddle shape) that faces downward, the finger is likely to come into close contact with the constricted surface, and the user is likely to recognize a rotation angle through a tactile sense of the finger. Since the saddle shape has a steep front slope, the finger is likely to be caught on the front slope. Therefore, it is easy to apply forward kinetic energy to the operation unit from the finger.

An insertion part (a head and a shaft) is covered with a probe cover. In a cover-mounted state, an opening portion of the cover reaches the operation unit. Usually, a portion (neck portion) of the cover close to the opening portion is fixed to the constricted portion by an elastic band. In the fixed state, since the bulged portion is present on a front side of the constricted portion, the fixed state is likely to be maintained.

In general, a hyperbolic paraboloid is given as a representative example of the saddle shape. The saddle shape in the embodiment has symmetry (line symmetry) as viewed in the z direction, but has asymmetry (non-line symmetry) as viewed in the x direction. That is, as viewed in the x direction, the front slope is steeper than the rear slope. In other words, an average inclined angle of the front slope with respect to the z direction is larger than an average inclined angle of the rear slope with respect to the z direction.

In the embodiment, each xy cross-section of the operation unit is larger than every xy cross-section of the shaft. That is, the operation unit is thicker than the shaft as a whole. A transition portion is provided between the shaft and the operation unit. In the transition portion, the xy cross-section changes in the z direction. The operation unit has a bulged portion and a constricted portion. The bulged portion is a portion provided on a rear side of the transition portion and connected to the transition portion. The bulged portion bulges in the left-right direction and the downward direction. The constricted portion is a portion provided on a rear side of the bulged portion, connected to the bulged portion, and having a constricted surface.

With the above-described configuration, since the bulged portion and the constricted portion are connected to each other, the relative amount of depression in the constricted portion can be increased. In a case where the operation unit is viewed from the z direction and the dial plate of the clock is imaged, the constricted portion is depressed with respect to the operation unit, for example, over a downward angle range from at least 4 o'clock to 8 o'clock. In other words, the bulged portion is bulged with respect to the constricted portion, for example, over a downward angle range from at least 4 o'clock to 8 o'clock. However, the 12 o'clock direction is the upward direction, and the 6 o'clock direction is the downward direction.

In the embodiment, the maximum xy cross-section of the bulged portion is larger than every xy cross-section of the constricted portion. In other words, the maximum xy cross-section (or the maximum peripheral length) of the bulged portion is larger than any xy cross-section (or any peripheral length) of the constricted portion.

In the embodiment, the operation unit includes a rear portion provided on a rear side of the constricted portion. The rear portion is connected to the constricted portion and connected to the probe cable. The maximum xy cross-section of the bulged portion is larger than every xy cross-section of the rear portion. In other words, the maximum xy cross-section (or the maximum peripheral length) of the bulged portion is larger than any xy cross-section (or any peripheral length) of the rear portion.

In the embodiment, the ultrasound probe has a central axis parallel to the z direction. The constricted surface has a saddle point that is a point belonging to a yz cross-section including the central axis and is the highest point in the y direction. The front slope is a surface provided on a front side of the saddle point. The rear slope is a surface provided on a rear side of the saddle point. The front slope is a curved surface, and the rear slope is also a curved surface. As described above, the front slope is steeper than the rear slope as viewed in the x direction. Since the rear slope is a gentle slope, various ways of holding are allowed.

In the embodiment, the operation unit includes a pair of boundary lines that are a pair of ridges protruding to both sides in the x direction. The back and the belly are divided by the pair of boundary lines. A width of the operation unit in the x direction changes along the y direction. In a case where a position in the y direction at which the maximum width occurs is defined as a maximum width height, the pair of ridges are generated by a continuous change in the maximum width height along the z direction.

In the embodiment, the back and the belly are distinguished by the pair of boundary lines under a certain condition. Specifically, as viewed in the z direction, a portion above the pair of boundary lines in an upper angle range from 9 o'clock to 3 o'clock is the back. In a case where the pair of boundary lines are lowered beyond the upper angle range, the upper angle range may be defined as the back.

In the embodiment, each of the pair of boundary lines has an apex, a drop portion, and a slope portion. The apex is a highest point as viewed in the x direction. The drop portion is a portion extending forward from the apex and is a portion hanging down forward as viewed in the x direction. The slope portion is a portion extending rearward from the apex and is a portion inclined as viewed in the x direction. This configuration reflects the shape of the belly according to the embodiment.

In the embodiment, the saddle shape has a plurality of downwardly convex curves belonging to a plurality of xy cross-sections and a plurality of upwardly convex curves belonging to a plurality of yz cross-sections. Each of the plurality of downwardly convex curves has symmetry. Each of the plurality of upwardly convex curves has asymmetry.

In the embodiment, the back has a flat surface extending along the z direction. For example, a thumb comes into contact with the flat surface. A thickness (size in the y direction) of the constricted portion is smaller than a width (size in the x direction) of the constricted portion. That is, the constricted portion has a flat xy cross-section. In a case where a finger pad is placed on the constricted surface, a rotation angle of the ultrasound probe can be naturally recognized.

In the embodiment, the transition portion has a cutout and a protrusion. The cutout comprises a first surface facing forward and a second surface facing laterally. The protrusion is integrated with the first surface and the second surface and protrudes from the first surface and the second surface. With this configuration, the protrusion is easily cleaned.

In the embodiment, the protrusion includes a semi-cylindrical main body and a rounded front end portion connected to the main body. The protrusion is engaged with a recessed portion or an opening of the puncture adapter. With this configuration, the puncture adapter can be easily and reliably attached.

### (2) Details of Embodiment

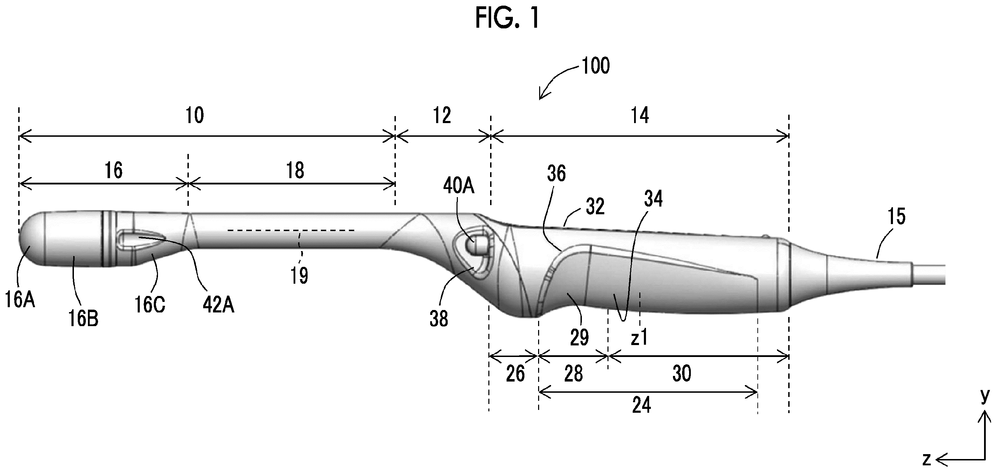

FIG. 1 shows an ultrasound probe 100 according to the embodiment. The shown ultrasound probe 100 is an intracavitary insertion-type ultrasound probe, and is inserted into, for example, a vagina. The configuration described below may be applied to other ultrasound probes.

In FIG. 1, a z direction is a front-rear direction, an x direction (a direction penetrating a paper surface) is a left-right direction, and a y direction is an up-down direction. The x direction, the y direction, and the z direction are orthogonal to each other. The front-rear direction is a direction of insertion, the left-right direction is a width direction, and the up-down direction is a thickness direction.

The ultrasound probe 100 includes an insertion part 10, a transition portion 12, and an operation unit 14. The insertion part 10 is a portion to be inserted into the body cavity. The transition portion 12 and the operation unit 14 are non-insertion parts. The insertion part 10 includes a head 16 and a shaft 18.

An ultrasound transducer is provided in the head 16. In the embodiment, a transducer array is provided as the ultrasound transducer. The transducer array transmits an ultrasound wave, and a reflected wave is received. The transducer array will be described in detail below. The head 16 includes a tip part 16A, an intermediate part 16B, and a rear end part 16C. In the rear end part 16C, a size of an xy cross-section changes along the z direction. A pair of grooves 42A is formed in the rear end part 16C.

The shaft 18 is connected to the head 16 and supports the head 16. The shaft 18 has a rod-like form that is elongated in the z direction. A central axis of the ultrasound probe 100 matches a central axis 19 of the shaft 18.

The transition portion 12 is connected to the shaft 18 and supports the shaft 18. In the transition portion 12, a size of an xy cross-section changes along the z direction. The transition portion 12 has a pair of cutouts 38. A pair of protrusions 40A is provided in the pair of cutouts 38. The pair of protrusions 40A and the pair of grooves 42A are structures for attaching a puncture adapter, which will be described below, to the ultrasound probe 100.

The operation unit 14 is connected to the transition portion 12, directly supports the transition portion 12, and indirectly supports the shaft 18. The operation unit 14 is a portion that is held or operated by a hand of a user (a doctor, an examination technician, or the like). The operation unit 14 has a bulged portion 26, a constricted portion 28, and a rear portion 30. The bulged portion 26 and the constricted portion 28 correspond to a front portion.

The operation unit 14 has a back 32 facing upward and a belly 34 facing downward. The operation unit 14 has a pair of ridges 36. The pair of ridges 36 is a pair of ridgelines that protrude to both sides in the x direction. The pair of ridges 36 is a pair of boundary lines that divide the back 32 and the belly 34.

The bulged portion 26 is a portion that is burged in the downward direction as viewed in the x direction. More specifically, the bulged portion 26 is burged in a left direction, a left-down direction, a downward direction, a right-down direction, and a right direction as viewed in the z direction. The bulged portion 26 is connected to the transition portion 12 and is also connected to the constricted portion 28. A maximum xy cross-section of the bulged portion 26 is larger than every xy cross-section of the shaft, is larger than every xy cross-section of the constricted portion 28, and is larger than every xy cross-section of the rear portion 30.

The constricted portion 28 is recessed in the upward direction as viewed in the x direction. The constricted portion 28 has a constricted surface 29 belonging to the belly. The constricted surface 29 has a downward saddle shape. The saddle shape will be described in detail below. In a case of holding or operating the operation unit 14, the constricted surface 29 is placed on a finger or the finger is wound around the constricted surface 29. In this case, since the bulged portion 26 is present on the front side of the finger, a force in the forward direction is easily transmitted from the finger to the bulged portion 26. Since the bulged portion 26 and the constricted portion 28 are connected to each other, a relative constriction amount of the constricted portion 28 is increased.

The rear portion 30 is connected to the constricted portion 28 and connected to a probe cable 15. The rear portion 30 is generally a rod-like or cylindrical portion. A grip part 24 is usually a portion that is touched by the user. The belly 34 extends over the entire grip part 24. z1 indicates a midpoint in the z direction in the operation unit 14. The constricted portion 28 is located on the front side of the midpoint z1.

The probe cable 15 includes a plurality of signal lines. The ultrasound probe 100 and an ultrasound diagnostic apparatus main body (not shown) are electrically connected to each other via the probe cable 15. The probe cable 15 includes an end part connected to the rear portion 30. The end part includes a cable boot having a conical shape. The operation unit 14 has a central axis that is slightly inclined with respect to the z direction.

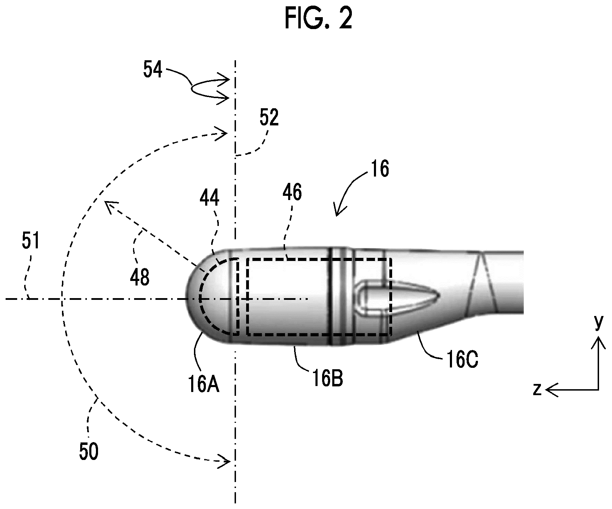

FIG. 2 shows the head 16. As already described, the head 16 includes a tip part 16A, an intermediate portion 16B, and a rear end part 16C. A transducer unit 44 is provided in the tip part 16A. The transducer unit 44 includes a convex-type transducer array. The transducer array consists of a plurality of transducers arranged in an arc shape.

A scanning mechanism 46 is provided in the intermediate portion 16B and the rear end part 16C. The scanning mechanism 46 includes a motor and a gear portion. The scanning mechanism 46 rotates the transducer unit 44 around a rotation axis 52. The transducer array forms an ultrasound beam 48. A beam scanning surface is formed by electronic scanning of the ultrasound beam 48 (see reference numeral 50). Reference numeral 51 indicates a central axis. The beam scanning surface is mechanically scanned by the scanning mechanism 46 (see reference numeral 54). A three-dimensional data acquisition space is formed by a combination of electronic scanning and mechanical scanning.

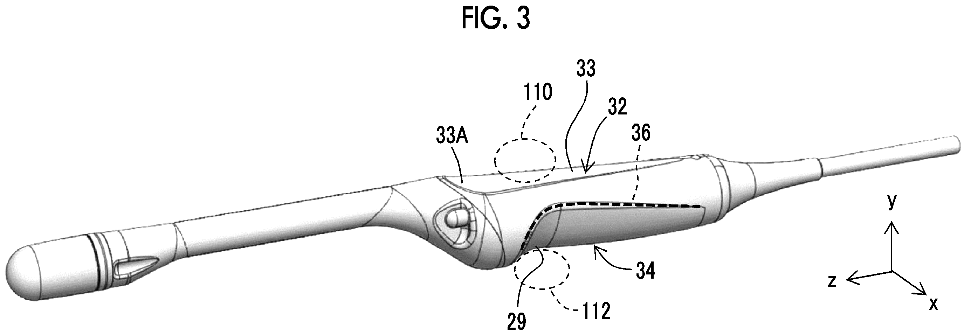

FIG. 3 shows an ultrasound probe according to an embodiment. As already described, the back 32 and the belly 34 are divided by a pair of ridges 36. The back 32 has a flat surface 33 facing upward. A front end portion 33A of the flat surface 33 is an inclined surface. The belly 34 has a constricted surface 29. For example, in a state in which the head is inserted into the body cavity, the constricted surface 29 is placed on a middle finger 112, and a thumb 110 is placed on the flat surface 33. The operation unit is pinched by two fingers. Of course, the operation unit may be held or operated by three or more fingers.

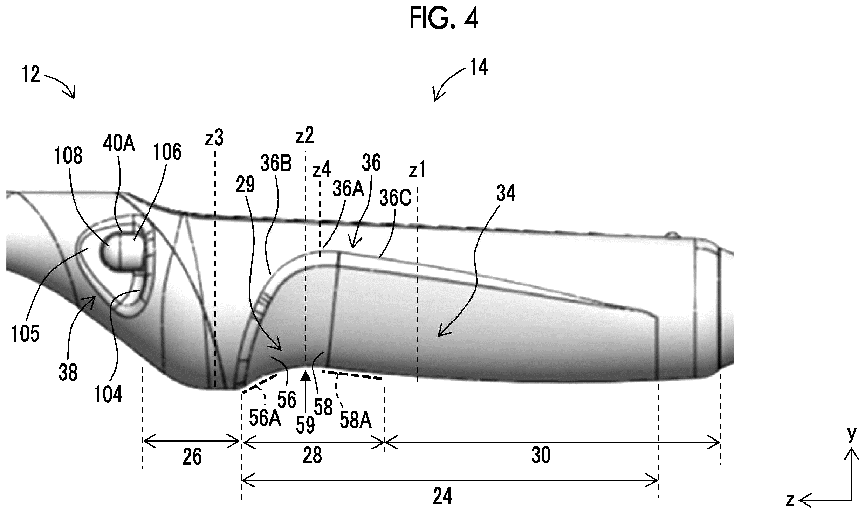

FIG. 4 shows the operation unit 14. As already described, the operation unit 14 has the bulged portion 26, the constricted portion 28, and the rear portion 30. In addition, the operation unit 14 has a pair of ridges 36. The lower side of the pair of ridges 36 is the belly 34. A surface facing upward on the upper side of the pair of ridges 36 is a back. The belly 34 extends over the entire grip part 24.

In the operation unit 14, a maximum width (maximum size in the x direction) is defined at each position in the z direction. In a case where a height (position in the y direction) at which the maximum width occurs is defined as a maximum width height, the maximum width height changes along the z direction. A movement trajectory of the maximum width height corresponds to the pair of ridges 36.

Each ridge 36 has an apex 36A that is the highest point in the y direction as viewed in the x direction, and has a drop portion 36B extending forward from the apex 36A and a slope portion 36C extending rearward from the apex 36A. The drop portion 36B hangs downward. The slope portion 36C gently descends.

The constricted portion 28 has a constricted surface 29. The constricted surface 29 has a downward saddle shape. The saddle shape has a curved front slope 56 and a curved rear slope 58. A highest point belonging to the constricted surface 29 and belonging to a median yz cross-section of the ultrasound probe (yz cross-section including a central axis of the ultrasound probe) is a saddle point 59. The front slope 56 is a slope on a front side of the saddle point 59, and the rear slope 58 is a slope on a rear side of the saddle point. As viewed in the x direction, the front slope 56 is steeper than the rear slope. In other words, as viewed in the x direction, an average inclined angle 56A of the front slope 56 is larger than an average inclined angle of the rear slope 58. A difference between a height of a lowest point of the bulged portion 26 and a height of the saddle point 59 is, for example, within a range of 2 to 10 mm or within a range of 3 to 8 mm.

In the z direction, z1 indicates a position of a midpoint of the operation unit 14, z2 indicates a position of the saddle point 59, z3 indicates a position where a maximum xy cross-section is generated in the operation unit 14, specifically, in the bulged portion 26, and z4 indicates a position of the apex 36A. The position z2 and the position z4 are close to each other. The positions are close to a position of a centroid in the z direction.

A pair of cutouts 38 are formed in the transition portion 12. Each cutout 38 has a first surface 104 facing forward and a second surface 105 facing laterally. The first surface 104 and the second surface 105 are orthogonal to each other. A pair of protrusions 40A is provided in the pair of cutouts 38. Each protrusion 40A is joined to the first surface 104 and the second surface 105, that is, integrated with the first surface 104 and the second surface 105. Each protrusion 40A consists of a main body 106 and a tip part 108. The main body 106 has a semicylindrical form. The tip part 108 has a rounded shape. The tip part 108 has a rounded shape, and specifically, the shape corresponds to 1/4 of a sphere.

In a case in which the insertion part is covered with the probe cover, the opening portion of the probe cover reaches the operation unit 14. The vicinity of the opening portion in the probe cover is fixed to the operation unit 14 by the elastic band. In this case, the elastic band is hooked on the constricted surface 29.

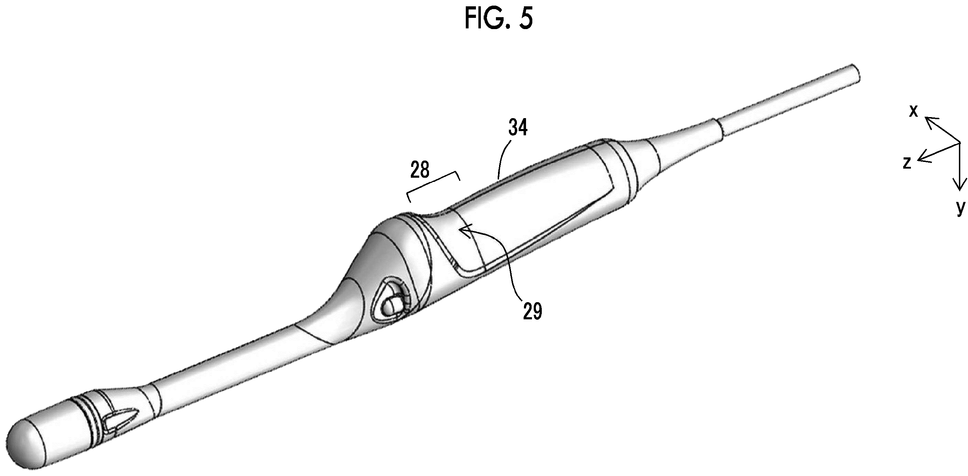

FIG. 5 shows the ultrasound probe that is turned over. The belly 34 faces upward. The constricted portion 28 has a constricted surface 29 having a saddle shape.

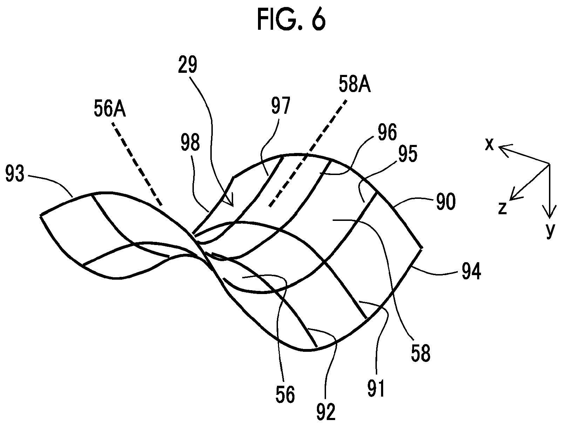

FIG. 6 schematically shows the upward constricted surface 29. As already described, the average inclined angle 56A of the front slope with respect to the z direction is larger than an average inclined angle 58A of the rear slope with respect to the z direction.

In a case in which a plurality of xy cross-sections passing through a plurality of positions in the z direction are defined for the constricted surface 29, a plurality of downwardly convex curves 90, 91, 92, and 93 appear on the plurality of xy cross-sections. However, in FIG. 6, each of the curves 90, 91, 92, and 93 is shown as an upwardly convex curve. Each of the curves 90, 91, 92, and 93 has line symmetry.

In a case in which a plurality of yz cross-sections passing through a plurality of positions in the x direction are defined for the constricted surface 29, a plurality of upwardly convex curves 94, 95, 96, 97, and 98 appear on the plurality of yz cross-sections. However, in FIG. 6, each of the curves 94, 95, 96, 97, and 98 is shown as a downwardly convex curve. Since the front slope is steeper than the rear slope, each of the curves 94, 95, 96, 97, and 98 does not have line symmetry.

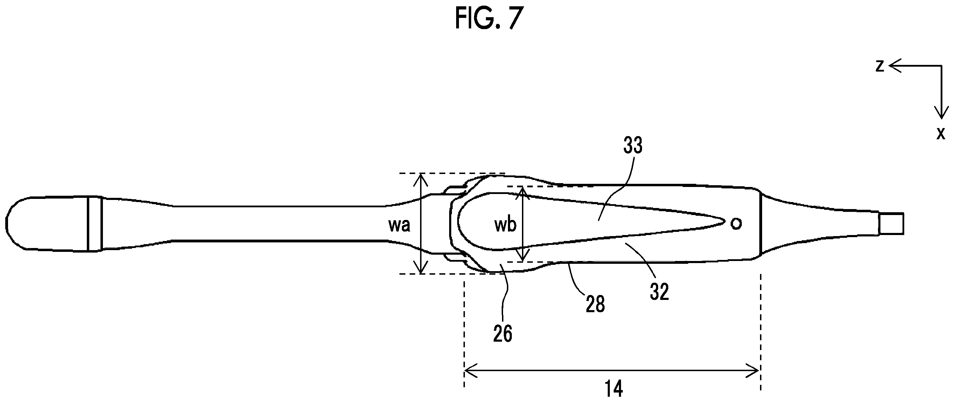

FIG. 7 shows an upper surface of the ultrasound probe. The operation unit 14 has a bulged portion 26 and a constricted portion 28. A width wa of the bulged portion 26 is larger than a width wb of the constricted portion 28. A back 32 has a flat surface 33. The flat surface 33 extends in the z direction. A width of the flat surface 33 gradually decreases from a distal end to a proximal end thereof.

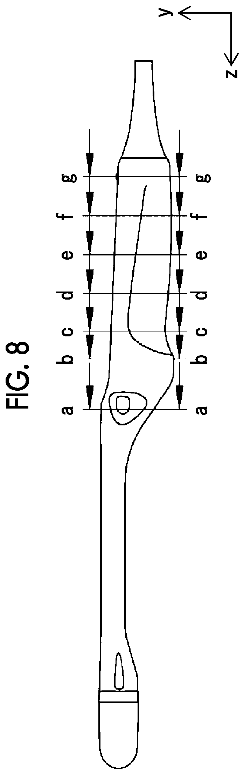

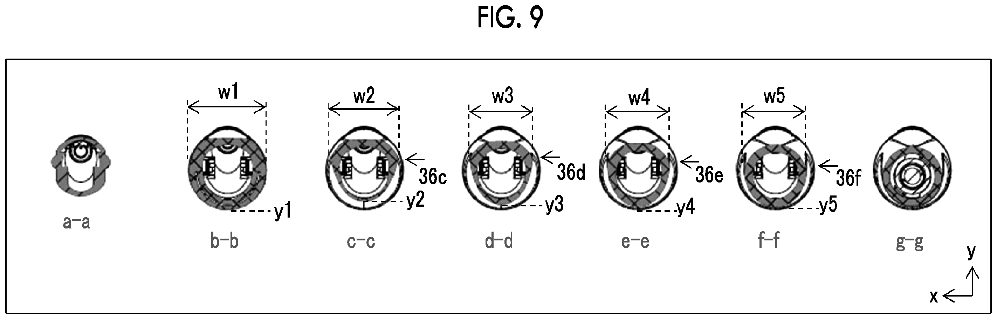

FIG. 8 shows a plurality of cross-section positions arranged in the z direction, which are set for the ultrasound probe according to the embodiment. FIG. 9 shows a plurality of xy cross-sections of the ultrasound probe. The plurality of xy cross-sections correspond to the plurality of cross-section positions.

In a cross-section taken along line b-b, a maximum width of the bulged portion is indicated by w1. A height of a lower end of the bulged portion is indicated by y1. In a cross-section taken along line c-c, a maximum width of the constricted portion is indicated by w2, and a maximum width height is indicated by 36c. A height of a lower end of the constricted portion is indicated by y2. y2 is higher than y1. A cross-section taken along line d-d is a cross-section in the vicinity of a distal end of a rear portion. A maximum width is indicated by w3, a maximum width height is indicated by 36d, and a height of a lower end is indicated by y3. In a cross-section taken along line e-e, a maximum width is indicated by w4, a maximum width height is indicated by 36e, and a height of a lower end is indicated by y4. In a cross-section taken along line f-f, a maximum width is indicated by w5, a maximum width height is indicated by 36f, and a height of a lower end is indicated by y5.

In a case where a dial plate of a clock is imaged on the cross-section taken along line c-c, the constricted portion is recessed at least over an angle range on a lower side from 4 o'clock to 8 o'clock. In other words, the bulged portion bulges at least over the angle range on the lower side from 4 o'clock to 8 o'clock.

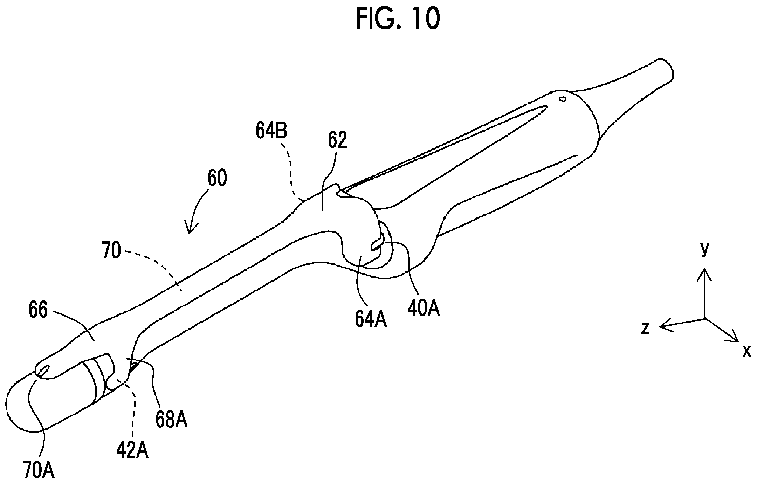

FIG. 10 shows an ultrasound probe to which a puncture adapter 60 is attached. The puncture adapter 60 holds or guides a biopsy needle. The puncture adapter 60 includes a tip part 66, a rear end part 62, and a main body 70. The tip part 66 includes a first arm 68A and a second arm (not shown). A part of the first arm 68A is inserted into the groove 42A. Similarly, a part of the second arm is inserted into a groove on an opposite side. The tip part 66 includes an opening 70A through which the biopsy needle passes.

The rear end part 62 includes a third arm 64A and a fourth arm 64B. The third arm 64A includes a depression, and a protrusion 40A is engaged with the depression. Similarly, the fourth arm 64B includes a depression, and a protrusion on an opposite side is engaged with the depression.

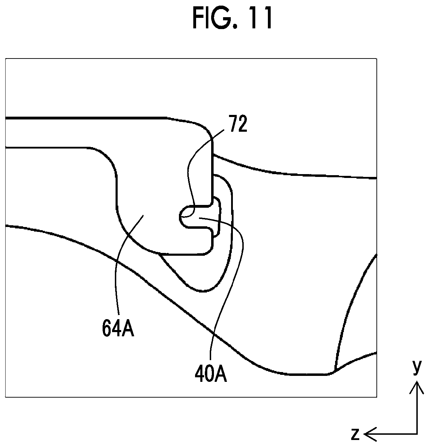

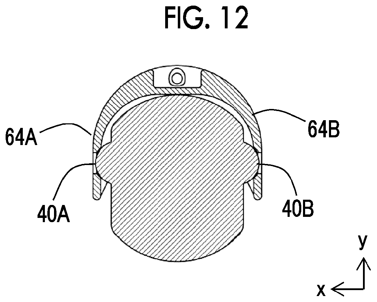

FIG. 11 shows an attachment portion. The protrusion 40A is inserted into a depression 72 of the third arm. The depression 72 includes a U-shaped groove. FIG. 12 shows a cross-section of the attachment portion. The third arm 64A is engaged with the protrusion 40A, and the fourth arm 64B is engaged with the protrusion 40B.

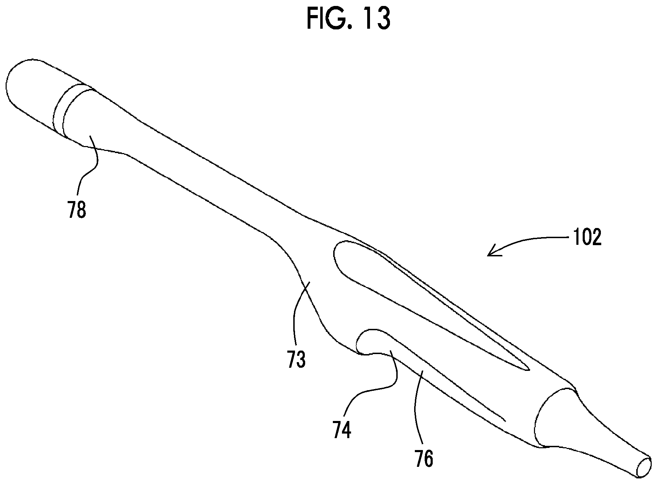

FIG. 13 shows an ultrasound probe 102 according to a modification example. The ultrasound probe 102 has a form close to the form of the ultrasound probe shown in FIG. 1. That is, the ultrasound probe 102 includes a transition portion 73, a constricted portion 74, and a rear portion 76. However, the transition portion 73 does not include a pair of cutouts. A head 78 also does not include a pair of grooves.

According to the above-described embodiment, the operability of the ultrasound probe can be improved. Specifically, according to the above-described embodiment, it is possible to provide an ultrasound probe comprising an operation unit that is easy to place on a finger, that is easy to hook a finger, or that can naturally recognize a rotation angle through a tactile sense of a finger.

### Explanation of References

10: insertion part
12: transition portion
14: operation unit
15: probe cable
16: head
18: shaft
24: grip part
26: bulged portion
28: constricted portion
29: constricted surface
30: rear portion
32: back
34: belly
36: ridge
100: ultrasound probe

## Claims

1. An ultrasound probe having a z direction that is a front-rear direction, an x direction that is a left-right direction orthogonal to the z direction, and a y direction that is an up-down direction orthogonal to the z direction and the x direction, the ultrasound probe comprising:
a head having an ultrasound transducer and inserted into a body cavity;
a shaft provided on a rear side of the head, supporting the head, and extending in the z direction; and
an operation unit provided on a rear side of the shaft and supporting the shaft,
wherein the operation unit has a back facing upward and a belly facing downward,
the belly has a constricted surface recessed upward as viewed in the x direction,
the constricted surface has a saddle shape including a front slope and a rear slope, and
the front slope is steeper than the rear slope as viewed in the x direction.

2. The ultrasound probe according to claim 1,
wherein each xy cross-section of the operation unit is larger than every xy cross-section of the shaft,
a transition portion is provided between the shaft and the operation unit, and
the operation unit has
a bulged portion provided on a rear side of the transition portion and connected to the transition portion, and
a constricted portion provided on a rear side of the bulged portion, connected to the bulged portion, and having the constricted surface.

3. The ultrasound probe according to claim 2,
wherein a maximum xy cross-section of the bulged portion is larger than every xy cross-section of the constricted portion.

4. The ultrasound probe according to either claim 2 or claim 3,
wherein the operation unit includes a rear portion provided on a rear side of the constricted portion,
the rear portion is connected to the constricted portion and connected to a probe cable, and
the maximum xy cross-section of the bulged portion is larger than every xy cross-section of the rear portion.

5. The ultrasound probe according to any one of claims 1 to 4,
wherein the ultrasound probe has a central axis parallel to the z direction,
the constricted surface has a saddle point that is a point belonging to a yz cross-section including the central axis and is a highest point in the y direction,
the front slope is a surface provided on a front side of the saddle point, and
the rear slope is a surface provided on a rear side of the saddle point.

6. The ultrasound probe according to any one of claims 1 to 5,
wherein the operation unit includes a pair of boundary lines that are a pair of ridges protruding to both sides in the x direction, and
the back and the belly are divided by the pair of boundary lines.

7. The ultrasound probe according to claim 6,
wherein each of the pair of boundary lines has
an apex that is highest as viewed in the x direction,
a drop portion that is a portion extending forward from the apex and hangs down forward as viewed in the x direction, and
a slope portion that is a portion extending rearward from the apex and is inclined as viewed in the x direction.

8. The ultrasound probe according to any one of claims 1 to 7,
wherein the saddle shape has
a plurality of downwardly convex curves belonging to a plurality of xy cross-sections, and
a plurality of upwardly convex curves belonging to a plurality of yz cross-sections.

9. The ultrasound probe according to any one of claims 1 to 8,
wherein the back has a flat surface extending along the z direction.

10. The ultrasound probe according to any one of claims 2 to 9,
wherein the transition portion includes
a cutout including a first surface facing forward and a second surface facing laterally, and
a protrusion integrated with the first surface and the second surface and protruding from the first surface and the second surface.

11. The ultrasound probe according to claim 10,
wherein the protrusion includes
a semi-cylindrical main body, and
a rounded front end portion connected to the main body.
